(19) Europäisches Patentamt
European Patent Office
Office européen des brevets

(11) EP 1 244 791 B1

(12) # EUROPÄISCHE PATENTSCHRIFT

(45) Veröffentlichungstag und Bekanntmachung des Hinweises auf die Patenterteilung:
**20.08.2008 Patentblatt 2008/34**

(21) Anmeldenummer: **00991641.2**

(22) Anmeldetag: **27.12.2000**

(51) Int Cl.:
***C12N 15/53*** (2006.01) ***C12N 9/04*** (2006.01)
***C12P 19/12*** (2006.01) ***C12P 19/24*** (2006.01)
***C07K 16/40*** (2006.01)

(86) Internationale Anmeldenummer:
**PCT/EP2000/013283**

(87) Internationale Veröffentlichungsnummer:
**WO 2001/048214 (05.07.2001 Gazette 2001/27)**

(54) **VERFAHREN ZUR HERSTELLUNG VON 6-O-ALPHA-D-GLUCOPYRANOSYL-D-SORBIT**

METHOD FOR PRODUCING 6-O-ALPHA-D-GLUCOPYRANOSYL-D-SORBITOL

PROCEDE DE PREPARATION DU 6-O-ALPHA-D-GLUCOPYRANOSYL-D-SORBITOL

(84) Benannte Vertragsstaaten:
**AT BE CH CY DE DK ES FI FR GB GR IE IT LI LU MC NL PT SE TR**

(30) Priorität: **24.12.1999 DE 19963126**

(43) Veröffentlichungstag der Anmeldung:
**02.10.2002 Patentblatt 2002/40**

(73) Patentinhaber: **SÜDZUCKER AKTIENGESELLSCHAFT**
**68165 Mannheim (DE)**

(72) Erfinder:
- **KUNZ, Markwart**
**67550 Worms (DE)**
- **MUNIR, Mohammad**
**67271 Kindenheim (DE)**
- **MATTES, Ralf**
**70619 Stuttgart (DE)**
- **KULBE, Klaus, Dieter**
**A-1180 Wien (AT)**

(74) Vertreter: **Schrell, Andreas et al**
**Gleiss Grosse Schrell & Partner**
**Patentanwälte Rechtsanwälte**
**Leitzstrasse 45**
**70469 Stuttgart (DE)**

(56) Entgegenhaltungen:
**US-A- 5 888 786**

- **SCHAUDER S ET AL: "POLYOL METABOLISM OF RHODOBACTER SPHAEROIDES: BIOCHEMICAL CHARACTERIZATION OF A SHORT-CHAIN SORBITOL DEHYDROGENASE" MICROBIOLOGY, SOCIETY FOR GENERAL MICROBIOLOGY, READING, GB, Bd. 141, 1995, Seiten 1857-1863, XP002914613 ISSN: 1350-0872**
- **REUTER R ET AL: "Purification and characterization of glucose-6-phosphate dehydrogenase from Pseudomonas W6." BIOMEDICA BIOCHIMICA ACTA. 1990, Bd. 49, Nr. 7, 1990, Seiten 539-546, XP008049074 ISSN: 0232-766X**
- **SCHNEIDER K-H ET AL: "SORBITOL DEHYDROGENASE FROM PSEUDOMONAS SP.: PURIFICATION, CHARACTERIZATION AND APPLICATION TO QUANTITATIVE DETERMINATION OF SORBITOL" ENZYME AND MICROBIAL TECHNOLOGY, STONEHAM, MA, US, Bd. 13, Nr. 4, April 1991 (1991-04), Seiten 332-337, XP009009183 ISSN: 0141-0229**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 244 791 B1

SEQUENZPROTOKOLL

<110> Südzucker AG mannheim/Ochsenfurt

<120> Verfahren zur Herstellung von 6-O-alpha-D-Glucopyranosyl-D-sorbit

<130> 15441Sdz

<140>
<141>

<160> 9

<170> PatentIn Ver. 2.1

<210> 1
<211> 262
<212> PRT
<213> Gluconobacter suboxydans

<400> 1

```
Met Ser Lys Lys Phe Asn Gly Lys Val Cys Leu Val Thr Gly Ala Gly
1               5                   10                  15

Gly Asn Ile Gly Leu Ala Thr Ala Leu Arg Leu Ala Glu Glu Gly Thr
            20                  25                  30

Ala Ile Ala Leu Leu Asp Met Asn Arg Glu Ala Leu Glu Lys Ala Glu
        35                  40                  45

Ala Ser Val Arg Glu Lys Gly Val Glu Ala Arg Ser Tyr Val Cys Asp
    50                  55                  60

Val Thr Ser Glu Glu Ala Val Ile Gly Thr Val Asp Ser Val Val Arg
65                  70                  75                  80

Asp Phe Gly Lys Ile Asp Phe Leu Phe Asn Asn Ala Gly Tyr Gln Gly
                85                  90                  95

Ala Phe Ala Pro Val Gln Asp Tyr Pro Ser Asp Asp Phe Ala Arg Val
            100                 105                 110

Leu Thr Ile Asn Val Thr Gly Ala Phe His Val Leu Lys Ala Val Ser
        115                 120                 125

Arg Gln Met Ile Thr Gln Asn Tyr Gly Arg Ile Val Asn Thr Ala Ser
    130                 135                 140

Met Ala Gly Val Lys Gly Pro Pro Asn Met Ala Ala Tyr Gly Ala Ser
145                 150                 155                 160

Lys Gly Ala Ile Ile Ala Leu Thr Glu Thr Ala Ala Leu Asp Leu Ala
                165                 170                 175

Pro Tyr Asn Ile Arg Val Asn Ala Ile Ser Pro Gly Tyr Met Gly Pro
            180                 185                 190

Gly Phe Met Trp Glu Arg Gln Val Glu Leu Gln Ala Lys Val Gly Ser
        195                 200                 205

Gln Tyr Phe Ser Thr Asp Pro Lys Val Val Ala Gln Gln Met Ile Gly
    210                 215                 220

Ser Val Pro Met Arg Arg Tyr Gly Asp Ile Asn Glu Ile Pro Gly Val
225                 230                 235                 240

Val Ala Phe Leu Leu Gly Asp Asp Ser Ser Phe Met Thr Gly Val Asn
                245                 250                 255

Leu Pro Ile Ala Gly Gly
            260
```

<210> 2
<211> 789

<212> DNA
<213> Gluconobacter suboxydans

<400> 2

```
atgtcgaaga agtttaacgg taaagtctgt ctggtcaccg gcgcgggtgg caatattggt 60
cttgcgaccg ccctccgtct ggcagaagag ggcacggcca tcgcccttct ggacatgaac 120
cgcgaggcgc tggaaaaggc ggaagcctcc gtccgtgaaa agggcgtcga agcccgctcc 180
tatgtctgtg acgtcacgtc cgaagaggcc gtgatcggga cggtggatag cgtggtccgg 240
gacttcggga agatcgactt cctgttcaac aatgccggct atcagggcgc cttcgccccc 300
gtgcaggact acccgtccga cgatttcgcg cgcgtgctga cgatcaacgt caccggtgcc 360
ttccacgtcc tcaaagccgt ttcgcgccag atgatcacgc agaactacgg gcgcatcgtc 420
aacaccgcca gcatggccgg tgtgaaggga ccgccaaaca tggccgccta tggtgcgtcc 480
aagggcgcca tcatcgccct gaccgaaacg gccgcgcttg accttgcccc ctacaacatc 540
cgtgtgaacg ccatcagccc cggttacatg gggcccggtt tcatgtggga gcgtcaggtc 600
gagcttcagg ccaaggtcgg aagccagtat ttctccaccg atcccaaggt cgtggcccag 660
cagatgatcg gcagcgttcc gatgcgccgc tatggcgaca tcaacgagat cccgggcgta 720
gtagcgttcc tgctggggga tgattccagc ttcatgacgg gggtgaacct gccgattgct 780
ggcggttga                                                         789
```

<210> 3
<211> 29
<212> PRT
<213> Gluconobacter suboxydans

<400> 3

```
Lys Lys Phe Asn Gly Lys Val Cys Leu Val Thr Gly Ala Gly Gly Asn
 1               5                  10                  15

Ile Gly Leu Ala Thr Ala Leu Arg Leu Ala Glu Glu Gly
            20                  25
```

<210> 4
<211> 14
<212> PRT
<213> Gluconobacter suboxydans

<400> 4

```
Val Leu Thr Ile Asn Val Thr Gly Ala Phe His Val Leu Lys
 1               5                  10
```

<210> 5
<211> 12
<212> PRT
<213> Gluconobacter suboxydans

<400> 5

Gly Pro Pro Asn Met Ala Ala Tyr Gly Ala Ser Lys
1 5 10

<210> 6
<211> 13
<212> PRT
<213> Gluconobacter suboxydans

<400> 6

Val Val Ala Gln Gln Met Ile Gly Ser Val Pro Met Arg
1 5 10

<210> 7
<211> 17
<212> PRT
<213> Gluconobacter suboxydans

<400> 7

Val Asn Ala Ile Ser Pro Gly Tyr Met Gly Pro Gly Phe Met Ser Glu
1 5 10 15

Arg

<210> 8
<211> 11
<212> PRT
<213> Gluconobacter suboxydans

<400> 8

Val Gly Ser Gln Tyr Phe Ser Thr Asp Pro Lys

1 5 10

<210> 9
<211> 21
<212> PRT
<213> Gluconobacter suboxydans

<400> 9

Ser Tyr Val Cys Asp Val Thr Ser Glu Glu Ala Val Ile Gly Thr Val
1 5 10 15

Asp Ser Val Val Arg
20

## Beschreibung

Die vorliegende Erfindung betrifft ein Verfahren zur enzymatischen Herstellung von 6-0-α-D-Glucopyranosyl-D-sorbit (im folgenden 1,6-GPS) aus Isomaltulose oder Saccharose.

Bekannt sind Verfahren zur Herstellung von 1,6-GPS aus Saccharose, die eine enzymatische Umwandlung von Saccharose zu Isomaltulose und anschließend eine chemische Hydrierung der erhaltenen Isomaltulose zu den beiden Stereoisomeren 1,6-GPS und 1-0-α-D-Glucopyranosyl-D-mannit (im folgenden 1,1-GPM) umfassen.

Aus der DE 44 14 185 C1 ist eine Saccharose-Isomerase bekannt, die die Umwandlung von Saccharose zu Isomaltulose katalysiert.

Schiweck (alimenta 19 (1980), 5-16) offenbart die enzymatische Umsetzung von Saccharose zu Isomaltulose und die anschließende Hydrierung der Isomaltulose an Raney-Nickel-Katalysatoren zu Palatinit® (auch Isomalt genannt), einem nahezu äquimolaren Gemisch aus 1,6-GPS und 1,1-GPM. Die Druckschrift beschreibt die Herstellung von Isomaltulose durch Transglucosidierung von Saccharose durch *Protaminobacter rubrum.* Außerdem wird offenbart, wie in Gegenwart von Raney-Nickel-Katalysatoren die durch Mikroorganismen gewonnene Isomaltulose mittels Hydrierung in 1,6-GPS und 1,1-GPM umgewandelt und anschließend durch Verdampfungs- und Kühlungskristallisationen angereichert werden kann.

Die DE 197 01 439 A1 offenbart Verfahren zur Hydrierung von Isomaltulose mittels eines trägergebundenen Nickelkatalysators, wobei Gemische aus 1,6-GPS und 1,1-GPM erhalten werden.

Die DE 197 05 664 A1 beschreibt ein Verfahren zur Herstellung von 1,6-GPS- oder 1,1-GPMangereicherten Gemischen. In dieser Druckschrift wird ein Verfahren offenbart, bei dem 1,6-GPS-und/oder 1,1-GPM-angereicherte Gemische aus hydrierter Isomaltulose oder aus Gemischen, die hydrierte Isomaltulose enthalten, hergestellt werden. Mittels dieses Verfahrens ist es möglich, 1,6-GPS rein herzustellen, indem 1,6-GPS-reiche Mutterlauge unter bestimmten Bedingungen aufkonzentriert und kühlungskristallisiert wird.

Die DE 195 23 008 A1 offenbart ein Verfahren zur Herstellung von 1,1-GPM und 1,6-GPS. Das Dokument beschreibt, wie bei Drücken von unter 50 Atmosphären verschiedene Katalysatoren so zum Einsatz kommen, dass durch die Hydrierung von Isomaltulose Gemische aus 1,6-GPS und 1,1-GPM erhalten werden. Die offenbarten Katalysatoren, an denen Isomaltulose umgesetzt wird, enthalten Ruthenium, Nickel und deren Mischungen.

Aus der EP 0 625 578 B1 gehen Verfahren zur Gewinnung von 1,1-GPM und 1,6-GPS-haltigen Zuckeralkoholgemischen durch enzymatische Umlagerung von Saccharose zu Isomaltulose und Trehalulose sowie anschließende Hydrierung zu 1,1-GPM, 1,6-GPS und 1-0-α-D-Glucopyranosyl-D-sorbit (1,1-GPS) hervor.

Die Verfahren nach dem Stand der Technik werden vor allem aus zwei Gründen als nachteilig angesehen. Zum einen können mit den bisherigen Methoden nur Gemische aus 1,6-GPS und 1,1-GPM gewonnen werden, aus denen dann mittels aufwendiger chemischer und physikalischer Trennverfahren der interessierende Stoff (1,6-GPS oder 1,1-GPM) isoliert werden muss. Zum anderen ist es nicht möglich, das gesamte Verfahren der Umwandlung von Saccharose zu den gewünschten Endprodukten in einem einzigen Verfahrensschritt durchzuführen. Im Stand der Technik sind also recht komplizierte Verfahrensabfolgen für die Herstellung von 1,6-GPS aus Saccharose erforderlich, wobei verschiedene physikalische, chemische und biologische Verfahrensschritte in verschiedenen Reaktoren zum Einsatz kommen. Für die Hydrierung der Isomaltulose sind nach gegenwärtigem Stand der Technik Katalysatoren, spezielle Hydrierreaktoren und technischer Wasserstoff notwendig. Ein wesentlicher Nachteil der bekannten Hydrierung von Isomaltulose zu 1,6-GPS und 1,1-GPM besteht also in dem erforderlichen technischen Aufwand. Oft sind für die Hydrierung Drücke von ungefähr 50 bis über 100 Atmosphären erforderlich, was einen spezifischen apparativen Aufbau erzwingt. Da die erhaltenen Produkte in der Regel in der Lebensmittelindustrie zum Einsatz kommen, muss der Verfahrensgang außerdem so gewählt sein, dass kein toxisches Material, zum Beispiel der Katalysatoren, in die Endprodukte der Hydrierungsreaktion gelangt. Das gebildete Produkt ist ein Gemisch aus den wesentlichen Bestandteilen 1,6-GPS und 1,1-GPM, aus dem der interessierende Stoff jeweils durch chemische und chemisch-physikalische Reinigungsmethoden abgetrennt werden muss. Um chemisch reines 1,6-GPS oder 1,1-GPM zu gewinnen, sind nach der erfolgten chemischen Hydrierung also aufwendige Anreicherungs- und Isolierungsprozeduren erforderlich. Die Ausbeute dieser Verfahrensschritte ist häufig nicht befriedigend.

Das der vorliegenden Erfindung zugrundeliegende technische Problem besteht also darin, ein Verfahren bereitzustellen, das eine einfache, kostengünstige und selektive Herstellung von 1,6-GPS aus Isomaltulose ermöglicht.

Die vorliegende Erfindung löst dieses technische Problem durch die Bereitstellung eines Verfahrens zur enzymatischen Herstellung von 1,6-GPS aus Isomaltulose, wobei die Isomaltulose in eine wässrige Reaktionslösung enthaltend eine Sorbitol-Dehydrogenase(SDH)-Aktivität aufweisende Einheit gegeben, inkubiert und 1,6-GPS aus der Reaktionslösung gewonnen wird. Die Erfindung ermöglicht es also, Isomaltulose biochemisch oder enzymatisch zu 1,6-GPS mittels einer SDH-Aktivität aufweisenden Einheit zu hydrieren. Die erfindungsgemäß eingesetzte SDH-Aktivität aufweisende Einheit reduziert Isomaltulose spezifisch zu 1,6-GPS. Die SDH-Aktivität aufweisende Einheit ist dadurch gekennzeichnet, dass sie in der Lage ist, Isomaltulose oder diese enthaltende Gemische enzymatisch zu 1,6-GPS oder dieses enthaltende Gemische umzusetzen. Der Vorteil der enzymatischen Umwandlung von Isomaltulose zu 1,6-GPS liegt neben der dadurch ermöglichten einfachen Verfahrensweise in der hohen Reaktions-, Substrat- und Stereospezifität, in der Einheitlichkeit des Reaktionsproduktes, in der Einsparung von Energie und Rohstoffen und in ihrer Umweltverträglichkeit.

Die Erfindung betrifft also die überraschende Lehre, dass mittels eines spezifischen enzymatischen Verfahrens aus Isomaltulose ganz gezielt 1,6-GPS hergestellt werden kann. Erfindungsgemäß wird in eine wässrige Reaktionslösung, die eine Sorbitdehydrogenase (SDH)-Aktivität aufweisende Einheit enthält, das Edukt Isomaltulose gegeben und solange inkubiert, bis 1,6-GPS gebildet wird und aus der Reaktionslösung mittels herkömmlicher Verfahren, zum Beispiel Kristallisation, gewonnen werden kann.

Im Zusammenhang mit der vorliegenden Erfindung wird unter einer wässrigen Reaktionslösung ungepuffertes oder mittels herkömmlicher Puffersysteme gepuffertes Wasser verstanden, in dem je nach Ausgangs- und Zielbedingungen Zusatzstoffe wie Stabilisatoren, Indikatorsysteme, Reduktionsäquivalente, Regenerationsmittel, Nährmediumsbestandteile, Salze, Zucker, Zuckeralkohole etc. enthalten sind.

In einer bevorzugten Ausführungsform der Erfindung ist die SDH-Aktivität aufweisende Einheit das Enzym SDH. Für die erfindungsgemäße enzymatische Umsetzung von Isomaltulose zu 1,6-GPS ist es möglich, die SDH-Aktivität aufweisende Einheit, also das Enzym SDH, in gereinigter, angereicherter und/oder isolierter Form in die Reaktionslösung einzubringen, wobei das Enzym zum Beispiel natürlichen Ursprungs sein kann. Selbstverständlich kann die erfindungsgemäß eingesetzte SDH auch eine rekombinant hergestellte erfindungsgemäße SDH aus einem gentechnisch veränderten Organismus sein. Es ist vorteilhaft, isolierte SDH dann zu verwenden, wenn Untersuchungen zur Kinetik des Umsatzes von Isomaltulose zu 1,6-GPS zwecks Optimierung dieser Reaktion erfolgen sollen. Insbesondere alle Fragestellungen, die sich mit dem chemischen Gleichgewicht der Umsetzung von Isomaltulose zu 1,6-GPS beschäftigen, erfordern es, mit isolierten Enzymen zu arbeiten, da sonst Stöchiometrie, Dissoziationskonstanten, Fragen zur Hemmung oder Aktivierung des Enzyms, Probleme der Kooperativität, der Initialgeschwindigkeit, der Konformation, der Liganden und aller anderen Probleme der Enzymkinetik und der interessierenden Bindungsdaten nicht präzise genug analysiert werden können. Vor allem um die Reaktion gezielt zu beeinflussen, ist es vorteilhaft, dass die SDH-Aktivität aufweisende Einheit in isolierter Form vorliegt, da sonst nicht ausgeschlossen werden kann, dass durch Wechselwirkungen mit anderen Bestandteilen der Reaktionslösung die Resultate der pH-Wert-, Ionenkonzentration- und Temperatur-Modifikationen nicht reproduzierbar sind, da gereinigte, partiell gereinigte und an Zellen gebundene Enzyme in der Analytik divergierende Werte zeigen.

Es gibt demgemäß auch ein Verfahren zur partiellen oder vollständigen Isolierung einer SDH aus Mikroorganismen, insbesondere aus Mikroorganismen der Gattung Gluconobacter, besonders bevorzugt der Art *Gluconobacter suboxidans,* wobei in einem ersten Schritt die Mikroorganismen mittels üblicher Verfahren aufgeschlossen und zu einem Rohextrakt homogenisiert werden, in einem zweiten Schritt eine Separation des Rohextraktes mittels Anionentauscherchromatografie, in einem dritten Schritt eine Gelfiltration und in einem vierten Schritt eine Farbstoff-Affinitätschromatografie stattfindet. Das nach der Farbstoff-Affinitätschromatografie erhaltene partiell aufgereinigte Enzym kann mittels Chromatofocussierung oder in einer anderen bevorzugten Ausgestaltung mittels einer weiteren Farbstoff-Affinitätschromatografie mit anschließender Affinitätselution mit mindestens einem Reduktionsäquivalent zur Homogenität aufgereinigt werden.

Es gibt auch ein Verfahren zur Isolierung von SDH aus einem Mikroorganismus, wobei der Mikroorganismus in einem ersten Verfahrensschritt aufgeschlossen und zu einem Rohextrakt homogenisiert, in einem zweiten Verfahrensschritt der erhaltene Rohextrakt einer Anionentauscherchromatograpie, in einem dritten Verfahrensschritt einer ersten Farb-Liganden-Affinitäts-chromatographie und in einem vierten Verfahrensschritt eine zweiten Farb-Liganden-Affinitäts-chromatographie unterzogen wird. Besonders bevorzugt wird nach der ersten und vor der zweiten Farb-Liganden-Affinitätschromatographie eine Ultrafiltration durchgeführt. Besonders bevorzugt wird im Anschluss an das vorgenannte Verfahren, also nach der zweiten Farb-Liganden-Affinitätschromatographie eine Affinitätselution der reinen SDH durchgeführt, wobei dazu ein Reduktionsäquivalent, insbesondere NADH, eingesetzt wird.

In einer weiteren bevorzugten Ausführungsform ist die SDH-Aktivität aufweisende Einheit ein SDHenthaltender toter oder lebender Mikroorganismus oder ein Rohextrakt oder Homogenat davon. Die erfindungsgemäß in bevorzugter Weise vorgesehene Umsetzung mit toten, besonders bevorzugt getrockneten, Mikroorganismen erfolgt in einer bevorzugten Ausführungsform der vorliegenden Erfindung nach der Rehydratisierung dieser in Gegenwart einer SDH-Enzymlösung nach Standardmethoden zum Beispiel unter Zusatz von Tannin und/oder Glutaraldehyd und ist deshalb vorteilhaft, da das lösliche Enzym den Mikroorganismus in hoher Konzentration fest umlagert. Selbstverständlich kann die Rehydratisierung auch mit Wasser durchgeführt werden oder aber ganz auf eine gesonderte Rehydratisierung verzichtet werden, so dass die toten Organismen direkt in die wässrige Reaktionslösung eingebracht werden. Die erfindungsgemäß auch vorgesehene Umsetzung von Isomaltulose mit vitalen Mikroorganismen hat unter anderem den Vorteil, dass sie mit verhältnismäßig geringem Aufwand erfolgen kann.

Bioreaktoren, in denen Mikroorganismen zum Einsatz kommen, sind in der Regel überall einsetzbar und erfordern im Vergleich zu den chemischkatalytischen Umsetzungen von Isomaltulose zu 1,6-GPS einen geringeren Energie- und Wartungsaufwand. Selbstverständlich müssen die Reaktionsbedingungen (pH-Wert, Ionenkonzentration, Sauerstoff/Kohlendioxidbedarf, Spurenelemente, Temperaturen und ähnliches) so gewählt sein, dass die Mikroorganismen zu einer optimalen Umsetzung von Isomaltulose zu 1,6-GPS befähigt sind. Unter diesen Verfahrensbedingungen kann die SDH im natürlichen Mikro-Milieu -also innerhalb der Zelle- eine höhere Stabilität und Effektivität aufweisen als das isolierte Enzym. Außerdem kann unter geeigneten Bedingungen eine Zellvermehrung und somit eine Erhöhung der SDH-Konzentration möglich sein. Gegenüber dem Stand der Technik, gemäß dem mit aufwendig herzustellenden Katalysatoren und technischem Wasserstoff gearbeitet werden muss, bedeutet die enzymatische Umsetzung mittels Mikroorganismen also einen bedeutenden Vorteil, was Zuverlässigkeit, Automatisierung und Einfachheit sowie Qualität und Ausbeute des Endprodukts des Verfahrens angeht.

Erfindungsgemäß ist es in bevorzugter Ausführungsform der Erfindung vorgesehen, bestimmte Zellkompartimente oder Teile davon voneinander zu trennen oder zu vereinigen. Kohlenhydratstrukturen, Lipide oder Proteine und/oder Peptide sowie Nucleinsäuren, die in der Lage sind, die SDH-Aktivität aufweisende Einheit beziehungsweise das Enzym positiv oder negativ zu beeinflussen, können so vereinigt oder getrennt werden. Um diese Beeinflussung zu umgehen oder gezielt zu nutzen, werden aus den Mikroorganismen Rohextrakte hergestellt. Dies kann mittels Beschallung, durch Pressen, Homogenisieren, Mixen mit Glaskugeln, durch Detergenzien, Einfluss elektrischer Felder, mechanisches Bearbeiten in flüssigem Stickstoff oder durch enzymatischen Verdau der Zellmembran und -wände erfolgen. In einem weiteren Schritt kann in bevorzugter Ausführungsform der gewonnene Extrakt zentrifugiert werden, um die erfindungsgemäße Umsetzung mit dem Überstand oder dem Sediment durchzuführen. Die Herstellung des Rohextraktes hat weiterhin den Vorteil, dass mittels einfacher Messung der Extinktion das Verhältnis von Nucleinsäuren zu Enzym bestimmt werden kann. Unter bestimmten Verfahrensbedingungen kann es nämlich notwendig sein, die Nucleinsäuren abzutrennen, da diese Komplexe mit der SDH-Aktivität aufweisenden Einheit eingehen können und so die Effizienz der Umwandlung von Isomaltulose zu 1,6-GPS herabsetzen. Die Herstellung eines Rohextraktes im Sinne der Erfindung schließt auch alle Maßnahmen ein, die den Rohextrakt stabilisieren, beziehungsweise in seiner katalytischen Wirkung optimieren. Hierzu kann der Zusatz von Protaminsulfat, Manganchlorid und Lysozym ebenso gehören wie die Veränderung der Ionenstärke und alle Maßnahmen, die dazu dienen, die Protease-Aktivität des erfindungsgemäßen Rohextraktes so zu modifizieren, dass die SDH-Aktivität aufweisende Einheit stabilisiert und in ihrer Wirkung optimiert wird.

In einer weiteren bevorzugten Ausführungsform ist der SDH enthaltende Mikroorganismus oder der Organismus, aus dem die vollständig oder partiell gereinigte SDH stammt, ein Organismus der Gattung *Gluconobacter*, insbesondere *Gluconobacter suboxidans*, besonders bevorzugt *Gluconobacter suboxidans* DSM 2003. Insbesondere in der erfindungsgemäß bevorzugt vorgesehenen Kombination mit dem Coenzym-Regenerationssystem SDH/FDH (Formiat-Dehydrogenase) ist mit den genannten Stämmen eine sehr gute Ausbeute an 1,6-GPS zu erzielen. Jedoch ist die enzymatische Umsetzung durch Mikroorganismen keinesfalls auf diese beiden Stämme beschränkt. Sämtliche Organismen, insbesondere Mikroorganismen, die in der Lage sind, Isomaltulose in einem oder mehreren Schritten zu 1,6-GPS umzusetzen, können erfindungsgemäß eingesetzt werden, zum Beispiel auch Pilze, Hefen, Zellkulturen etc.. Dazu können Mutanten, gentechnisch geänderte oder sonstwie hergestellte oder isolierte Abwandlungen der vorgenannten Organismen zählen, zum Beispiel Organismen, die aufgrund des Einführens einer SDH-codierenden Nucleotidsequenz SDH-Aktivität aufweisen. Bevorzugt sind Organismen, die auch eine Saccharose-Mutase-Aktivität aufweisen, so dass mittels eines einzigen Organismustyps Saccharose enzymatisch zu 1,6-GPS umgewandelt werden kann.

In einer weiteren bevorzugten Ausführungsform sind die erfindungsgemäßen SDH-Aktivität aufweisenden Einheiten, insbesondere Mikroorganismen, Rohextrakte, Teile davon und/oder die angereicherten oder isolierten Enzyme immobilisiert. Durch Immobilisierung werden Enzyme, Zellorganellen und Zellen in einen unlöslichen und reaktionsraumbegrenzten Zustand versetzt. Erfindungsgemäß wird die SDH-Aktivität aufweisende Einheit unter Bedingungen, unter denen sie möglichst hohe SDH-Enzymaktivitäten aufweist, immobilisiert. Erfindungsgemäß ist auch der Einschluss von vitalen Zellen in Polymere vorgesehen. Die Immobilisierung kann durch (i) Bindung wie auch mittels (ii) Einschluss erfolgen. Bei einer Bindungs-Immobilisierung der SDH-Aktivität aufweisenden Einheit sind Trägerbindungen (ionisch oder kovalent) und Quervernetzungen (Vernetzung untereinander oder Vernetzung mit anderen Polymeren) vorhanden. Bei einer Einschluß-Immobilisierung der SDH-Aktivität aufweisenden Einheit wird der Einschluss in Gelstrukturen (Kugeln, Fasern und ähnliches) und in Membranen (Mikrokapseln und Membranreaktoren) vorgesehen. Unter Immobilisierung werden demgemäss alle Methoden verstanden, die zur Einschränkung der Beweglichkeit und Löslichkeit der SDH-Aktivität aufweisenden Einheit auf biologischem, chemischem oder physikalischem Wege dienen. Dies kann insbesondere durch Adsorption an inerte oder elektrisch geladene, anorganische oder organische Trägermaterialien erfolgen, wobei die anorganischen Materialien zum Beispiel poröse Gläser, Silicagel, Aluminiumoxid und Hydroxylapatit oder Metalloxide sind; die natürlichen Polymere können zum Beispiel Cellulose, Stärke, Dextran oder Agarose sein und die synthetischen Polymere können Polyacrylamid, Polyvinylalkohol, Nylon oder andere sein. Es ist erfindungsgemäß auch vorgesehen, die SDH-Aktivität aufweisende Einheit in ein dreidimensionales Netzwerk einzuschließen; in einer Ausführungsform der Erfindung kann dies Gelatine oder Agar sein. Weiterhin kann vorgesehen sein, die Immobilisierung mittels Quervernetzung mit bifunktionellen Agenzien wie Glutardialdehyd oder Benzidin durchzuführen. Es ist auch eine Mikroverkapselung der SDH-Aktivität aufweisenden Einheit möglich, die zur Eingrenzung des Reaktionsraums mit Hilfe von künstlichen oder biologischen Membranen führt. Die Erfindung erfasst auch eine Immobilisierung ohne Träger durch Flockung und "cross-linking" ebenso wie eine Coimmobilisierung lebender und/oder toter Zellen mit freien oder immobilisierten Enzymen. Auch die Molekulargewichtsvergrößerung beispielsweise durch die Bindung an Dextran dient der Immobilisierung im Sinne der Erfindung. Bei dem immobilisierten Enzym kann es sich um ein Enzym handeln, welches an Kompartimente eines Mikroorganismus oder an einem vollständig erhaltenen vitalen oder getrockneten Mikroorganismus gebunden ist, im Sinne der Erfindung kann immobilisiert jedoch auch bedeuten, dass das Enzym alleine oder das Enzym in Verbindung mit Kompartimenten des Organismus an einen Träger gebunden ist. Von Bedeutung ist, dass die beladene Matrix mit der Isomaltulose so in Berührung gelangen kann, dass die gewünschte enzymatische Reaktion zu 1,6-GPS abläuft. Selbstverständlich betrifft die Erfindung jedoch auch die Verwendung der genannten SDH-Aktivität aufweisenden Einheit in freier, das heißt nicht immobilisierter Form.

In einer weiteren bevorzugten Ausführungsform wird die erfindungsgemäße enzymatische Umwandlung mit oder ohne pH-Wert-Regulierung durchgeführt. Insbesondere, da in besonders bevorzugter Ausführungsform Reduktionsäquivalente in Form von Wasserstoff-überträgern zum Einsatz kommen, hat die Wahl des pH-Wertes Einfluss auf das Gleichgewicht der chemischen Reaktion wie auch auf die Gesamtausbeute an 1,6-GPS. Demgemäss lässt sich die enzymatische Umsetzung von Isomaltulose zu 1,6-GPS in die Reaktionen einteilen, bei denen keine Regulierung des pH-Wertes erfolgt und die, bei denen eine Regulierung des pH-Wertes erfolgt. Die Regulierung des pH-Wertes kann durch Zu- oder Abgabe von Stoffen erfolgen, die in der Lage sind, den pH-Wert anzuheben, zu senken oder zu stabilisieren. Erfindungsgemäß können Säuren oder Basen nach der Lowry-Brönsted- oder Lewis-Pearson-Definition verwendet werden, wobei Protonen, Hydronium-Ionen, Hydroxid-Ionen, oder freie Elektronenpaare abgegeben beziehungsweise zur Verfügung gestellt oder aufgenommen werden können. Beispielsweise kann eine Begasung der wässrigen Reaktionslösung mit $CO_2$ vorgesehen sein, die - als Einleitung einer Säure - eine Maßnahme zur pH-Wert-Regulierung ist. Es kann in vorteilhafter Weise angestrebt sein, dass sich der pH-Wert in einem Bereich einstellt, der als optimal für die Aktivität des Enzyms anzusehen ist. Dieses pH-Optimum kann durch verschiedene Puffer oder durch die Zugabe von pH-Wert-absenkenden oder erhöhenden Substanzen erfolgen. Insgesamt ist jedoch die pH-Wert-Regulierung keinesfalls auf chemische Verfahren wie zum Beispiel Komplexbildungsvorgänge, Titrationen oder ähnliches beschränkt. Jeder Eingriff in das System, der dazu führt, dass der pH-Wert in irgendeiner Weise reguliert oder stabilisiert werden kann, gilt im Sinne der Erfindung als Durchführung einer PH-Wert-Regulierung. In vorteilhafter Ausgestaltung der Erfindung liegt der pH-Wert der wässrigen Reaktionslösung bei etwa 6,0 bis 8,0, vorzugsweise bei pH 7,0.

In einer bevorzugten Ausführungsform der Erfindung werden der wässrigen Reaktionslösung neben der SDH-Aktivität aufweisenden Einheit auch Reduktionsäquivalente zugegeben, insbesondere dann, wenn gereinigte und isolierte SDH verwendet wird. Reduktionsäquivalente im Sinne der vorliegenden Erfindung sind Wasserstoffüberträger oder Elektronen-Carrier, die in Form von Coenzymen oder prosthetischen Gruppen vorliegen können. Solche Reduktionsäquivalente können beispielsweise $NAD^+$/NADH, NADP-/NADPH, FMN/$FMNH_2$ oder FAD/$FADH_2$ sein. Coenzyme und prosthetische Gruppen von Oxidoreduktionsreaktionen dienen als dissoziierbare und/oder fest an Proteine gebundene Wasserstoff- und/oder Elektronenüberträger. Als Reduktionsäquivalente können jedoch auch andere Coenzyme/prosthetische Gruppen dienen, die in der Lage sind, Wasserstoffatome bzw. Elektronen zu übertragen. Erfindungsgemäß bevorzugt sind insbesondere cyclische Tetrapyrrole, Glutathion, Liponsäure, Chinone, Eisen-Schwefel-Proteine, Flavo-Proteine, Flavin-Nucleotide und alle anderen, die während der enzymatischen Katalyse Protonen, Atome und/oder Elektronen beziehungsweise funktionelle Gruppen übertragen können.

In-bevorzugter Weise werden zur enzymatischen Herstellung von 1,6-GPS lebende SDH-Aktivität aufweisende Zellen verwendet, so dass sich die Zugabe von Reduktionsäquivalenten erübrigt.

In einer weiteren besonders bevorzugten Ausführungsform erfolgt die enzymatische Umsetzung von Isomaltulose zu 1,6-GPS in Gegenwart von Regenerationsmitteln wie Formiat-Dehydrogenase (FDH) und/oder Formiat, insbesondere dann, wenn gereinigte und isolierte SDH verwendet wird. Durch die Zugabe von SDH und FDH in die wässrige Reaktionslösung ist eine chemische Reaktion möglich, während der ständig Formiat zu $CO_2$ umgewandelt wird und Isomaltulose zu 1,6-GPS. Die Umwandlung des Formiats in $CO_2$ erfolgt über die FDH. Die bei dieser Oxidation von einem ebenfalls in der Reaktionslösung vorhandenen Reduktionsmittel, zum Beispiel $NAD^+$, aufgenommenen Wasserstoffatome werden bei der parallel ablaufenden Reduktion von Isomaltulose zu 1,6-GPS eingesetzt. Selbstverständlich können anstelle der FDH auch völlig andere Dehydrogenasen oder Enzyme mit anderen Substratspezifitäten als coenzym des Regenerierungssystems, also als Regenerationsmittel, in Frage kommen. Beispielsweise sind andere Carbonsäuren beziehungsweise deren Salze als Regenerationsmittel einsetzbar. Von Bedeutung ist, dass die zugegebenen Substanzen, insbesondere FDH/Formiat, in der Lage sind, mit wasserstoffüberträgern, Coenzymen oder prosthetischen Gruppen so zu reagieren, dass Elektronen und/oder Wasserstoffatome so übertragen werden, dass die kontinuierliche Bildung von 1,6-GPS möglich ist. Im Falle der Verwendung lebender Zellen als SDH-Aktivität aufweisender Einheit kann die Zugabe von Regenerationsmittels und Reduktionsäquivalenten unterbleiben.

In einer weiteren besonders bevorzugten Ausführungsform erfolgt die Umwandlung von Isomaltulose in 1,6-GPS im Rahmen eines kontinuierlichen Verfahrens. Das kontinuierliche Verfahren der Erfindung kann in einem durchströmten Reaktor durchgeführt werden, in dem mikrobielles Wachstum und somit die Produktbildung, die Synthese von 1,6-GPS, stattfindet. Auf diese Weise können sehr große Mengen von 1,6-GPS hergestellt werden. Durch die Verwendung des kontinuierlichen Verfahrens sind die Reinigungs- und Vorbereitungszyklen frei wählbar und nicht durch die Parameter von spezifischen Fermentatoren oder bestimmte physiologischen Eigenschaften der Mikroorganismen vorgegeben, da sich in kontinuierlichen Systemen das die Mikroorganismen umgebende Milieu nicht durch die verbrauchten Substrate und entstehenden Produkte ändert, weil die Produkte ständig abgeführt und die Substrate ständig zugeführt werden. Außerdem können die Bioreaktoren oder Anlagen im kontinuierlichen Verfahren viel kleiner und kostengünstiger ausgelegt werden. Unter einem kontinuierlichen Verfahren versteht man im Sinne der Erfindung auch alle ergänzenden Maßnahmen, die dazu dienen, die Gefahr der Infektion mit anderen Organismen, die sich durch die Substratdurchschleusung schnell im System ausbreiten können, zu verhindern. Dies kann sterile wie auch nicht-sterile Bedingungen einschließen. Die vorliegende Erfindung betrifft auch Verfahrensmaßnahmen und Mittel, die dazu dienen, die kontinuierliche Fermentation durch Extrembedingungen so zu gestalten, dass sie gegen mögliche Infektionen aufgrund der gewählten extremen Bedingungen (zum Beispiel puffern bei einem sehr niedriger pH-Wert oder Antibiotika-Einsatz) stabil sind. Dabei können erfindungsgemäß Abwandlungen oder Modifikationen der vorgenannten SDH-Aktivität aufweisenden Einheiten eingesetzt werden, die diesen infektionsfeindlichen Bedingungen angepasst sind, zum Beispiel Antibiotika-resistente Mikroorganismen. Unter einem kontinuierlichen Verfahren im Sinne der Erfindung kann jedoch auch jedes System wachsender Zellen und katalysierender Enzyme verstanden werden, dem einerseits Nährlösung zugeführt wird und aus dem andererseits Kulturlösung, einschließlich des enzymatisch gebildeten 1,6-GPS, abgezogen wird; hierbei kann es sich um homogene wie auch inhomogene Systeme handeln.

Die Erfindung betrifft selbstverständlich auch semikontinuierliche oder batchweise Verfahrensführungen.

In einer weiteren bevorzugten Ausführungsform wird die Isomaltulose, die der Ausgangsstoff für die Umwandlungsreaktion zu 1,6-GPS ist, durch enzymatische Umwandlung von Saccharose hergestellt. Die in dem erfindungsgemäßen Verfahren eingesetzte Isomaltulose kann durch enzymatische Umwandlung (Transglucosidierung) mittels gereinigter oder angereicherter Enzyme, Rohextrakte oder Mikroorganismen aus Saccharose hergestellt werden, die eine Saccharose-Mutaseaktivität aufweisen. Im Zusammenhang mit der vorliegenden Erfindung wird unter einer Saccharose-Mutase also ein Enzym verstanden, dass zur Isomerisierung von Saccharose zu Isomaltulose in der Lage ist und auch als Saccharose-Isomerase bekannt ist.

Beispiele für Zellen, die für ein Protein mit Saccharose-Mutase-Aktivität codierende Nucleotidsequenzen enthalten, sind insbesondere Mikroorganismen der Gattungen *Protaminobacter, Erwinia, Serratia, Leuconostoc, Pseudomonas, Agrobacterium, Klebsiella* und *Enterobacter.* Spezifische Beispiele für solche Mikroorganismen sind *Protaminobacter rubrum* (CBS 547,77), *Erwinia rhapontici* (NCPPB 1578), *Serratia plymuthica* (ATCC 15928), *Serratia marcescens* (NCIB 8285), *Leuconostoc mesenteroides* NRRL B-521f (ATCC 10830a), *Pseudomonas mesoacidophila* MX-45 (FERM 11808 beziehungsweise FERM BP 3619), *Agrobacterium radiobacter* MX-232 (FERM 12397 beziehungsweise FERM BP 3620), *Klebsiella subspecies* und *Enterobacter species.*

Spezifische Beispiele für Proteine mit Saccharose-Mutase Aktivität und dafür codierende Nucleinsäuren aus *P. rubrum, E. rhapontici, Enterobacter spec.* SZ62 und *P. mesoacidophila* sind in PCT/EP 95/00165 beschrieben.

Die genannten Zellen oder Proteine oder Nucleotidsequenzen aus diesen Zellen können zusammen mit einer SDH-Aktivität aufweisenden Einheit der vorliegenden Erfindung zur Herstellung von 1,6-GPS aus Saccharose verwendet werden, zum Beispiel, indem SDH-codierende Nucleotidsequenzen unter Kontrolle regulatorischer Einheiten in die vorgenannten Zellen eingeführt und exprimiert werden.

In einer besonders bevorzugten Ausführungsform wird die enzymatische Umsetzung von Saccharose zu Isomaltulose mittels, vorzugsweise immobilisierter, P. *rubrum-Zellen* (*Protaminobacter rubrum*) oder eines Zellaufschlusses oder Rohextraktes von P. *rubrum* oder mittels partiell oder vollständig gereinigter Saccharose-Mutase (auch Saccharose-Isomerase) durchgeführt. Diese Umsetzung geschieht bevorzugterweise zusammen in einer Reaktionslösung mit der SDH-abhängigen Umsetzung von Isomaltulose zu 1,6-GPS.

Die enzymatische Umlagerung von Saccharose zu Isomaltulose wird beispielsweise in der DE 195 23 560 A1 oder der DE 44 14 185 C1 beschrieben, die hinsichtlich der dort offenbarten Mikroorganismen, DNA-Sequenzen, Enzyme und Verfahren zur Herstellung von Isomaltulose durch enzymatische Umwandlung aus Saccharose vollständig in den Offenbarungsgehalt der vorliegenden Anmeldung einbezogen sind.

Die Immobilisierung von Saccharose-Mutase aufweisenden Zellen, zum Beispiel *P. rubrum,* von Zellaufschlüssen davon oder von dem isolierten Enzym (Saccharose-Mutase) dient der Einschränkung der Beweglichkeit und Löslichkeit der eingesetzten Biokatalysatoren auf biologischem, chemischem und/oder physikalischem Wege. Die Immobilisierung im Sinne der Erfindung kann durch unterschiedliche Methoden erfolgen, wie zum Beispiel die Bindung der Biokatalysatoren untereinander oder an Trägerstoffe, durch Fixieren im Netzwerk einer polymeren Matrix oder durch das Umschließen mit artifiziellen oder natürlichen Membranen, was selbstverständlich den Einsatz inverser Micellen einschließt. Durch die Immobilisierung der Zellen, der Zellaufschlüsse o-der/und der Saccharose-Mutase werden die so erzeugten Biokatalysatoren nicht nur wiederverwendbar, sie können vor allem nach oder während des Prozesses leicht abgetrennt werden, um sie gegebenenfalls durch andere Katalysatoren, die andere Reaktionen, wie die Umsetzung von Isomaltulose zu 1,6-GPS, katalysieren, zu ersetzen. Ein wesentlicher Vorteil ist, dass Zellen oder Enzyme durch die Immobilisierung in viel höheren lokalen Konzentrationen und vor allem in kontinuierlichen Durchflusssystemen eingesetzt werden können. Die Immobilisierung kann hierbei auf keramischen Trägern, auf Polymeren, auf verschiedenen Gelen und Gelatinen, durch Einschluss in Polyacrylamid oder andere Verfahren erfolgen.

In einer weiteren bevorzugten Ausführungsform erfolgt die Umwandlung von Saccharose zu Isomaltulose und von Isomaltulose zu 1,6-GPS in einem einzigen Verfahrensschritt, das heißt die beiden enzymatischen Umwandlungsreaktionen laufen gleichzeitig oder leicht zeitversetzt in ein und derselben wässrigen Reaktionslösung ab. Die Umwandlung von Saccharose zu 1,6-GPS kann deshalb in einem Verfahrensschritt erfolgen, da die beiden einzelnen Umwandlungsreaktionen enzymatisch unter gleichen oder ähnlichen Verfahrensbedingungen ablaufen können.

In einer besonders bevorzugten Ausführungsform erfolgt die Umsetzung von Saccharose zu 1,6-GPS in einem einzigen Bioreaktor (sogenannte "Eintopfreaktion").

Diese Ausgestaltungen der Erfindung umfassen die direkte enzymatische Herstellung von 1,6-GPS aus Saccharose, wobei sowohl für die Umsetzung von Saccharose zu Isomaltulose als auch von Isomaltulose zu 1,6-GPS vorgenannte nicht-immobilisierte und immobilisierte Zellen, Rohextrakte und/oder Enzyme zum Einsatz kommen können, die also jeweils SDH-und Saccharose-Mutase-Aktivitäten aufweisen.

Erfindungsgemäß ist die Umwandlung auch in einer Batch- und/oder Fed Batch-Fermentation (einer diskontinuierliche Fermentation) möglich, wobei das Nährmedium zu einem definierten Zeitpunkt beimpft wird und die Fermentation nach Verbrauch des limitierenden Substrates, was zum Beispiel Saccharose, andere Substrate oder Enzyme/Coenzyme sein können -oder zu einem anderen geeigneten Zeitpunkt- beendet wird. Die Reaktionsbedingungen können jedoch auch so gewählt sein, dass der limitierende Einfluss eines Substrates weitgehend ausgeschlossen wird. Batch-Reaktionen können in einem sogenannten geschlossenen System durchgeführt werden. Diese Systeme sind erfindungsgemäß auch als geschlossen aufzufassen, wenn der geschlossenen flüssigen Phase kontinuierlich Sauerstoff, Luft oder ein anderes Gas oder Gasgemisch zugeführt wird. In einem Batch-Verfahren ändert sich die Umgebung der Zellen ständig, da es zu einer Abnahme des Substrates und unter anderem zu einer Zunahme von 1,6-GPS und unter gewissen Umständen auch zu einer Zunahme der Zellkonzentration kommen kann. Das vorgenannte "Eintopfverfahren" kann im Sinne der Erfindung auch unter Einhaltung eines Fließgleichgewichtes -unter ständiger Abfuhr von 1,6-GPS- als kontinuierliche Enzymkatalyse durchgeführt werden.

In einer weiteren bevorzugten Ausführungsform beträgt die Temperatur der wässrigen Reaktionslösung bei der enzymatischen Umwandlung 20°C bis 40°C, insbesondere 25°C. Bei Temperaturen von 25°C weist die gereinigte SDH eine Stabilität von ca. einer Woche auf. Somit sind Temperaturen in diesem Bereich gut geeignet, um längerfristige enzymkatalytische Verfahren durchzuführen.

In einer weiteren bevorzugten Ausführungsform betrifft die Erfindung ein Verfahren zur Herstellung von 1,6-GPS aus Saccharose, wobei die Saccharose erst enzymatisch zu Isomaltulose umgewandelt und anschließend die Isomaltulose enzymatisch zu 1,6-GPS umgesetzt wird. Dieser gesamte Vorgang kann kontinuierlich, semi-kontinuierlich oder nichtkontinuierlich, in einem oder mehreren Verfahrensschritten und in einem oder mehreren Bioreaktoren ablaufen.

Es gibt auch ein Nucleinsäuremolekül codierend ein Enzym mit der Aktivität einer Sorbitol-Dehydrogenase (SDH), ausgewählt aus der Gruppe bestehend aus

a) Nucleinsäuremolekülen, die ein Protein codieren, das die unter SEQ ID Nr. 1 angegebene Aminosäuresequenz aufweist oder ein komplementärer Strang davon;

b) Nucleinsäuremolekülen, die die unter SEQ ID Nr. 2 dargestellte Nucleotidsequenz umfassen oder ein komplementärer Strang davon;

c) Nucleinsäuremolekülen, die mit einem unter a) oder b) genannten Nucleinsäuremolekül hybridisieren;

d) Nucleinsäuremolekülen, deren Nucleotidsequenz aufgrund der Degeneration des genetischen Codes von der Sequenz der unter b) oder c) genannten Nucleinsäuremoleküle abweicht.

Im Zusammenhang mit der vorliegenden Erfindung wird unter einem Enzym mit einer Sorbitol-Dehydrogenase-Aktivität ein Protein oder Peptid verstanden, das in der Lage ist, die Umwandlung von Isomaltulose zu 1,6-GPS zu katalysieren. Dabei kann die umzuwandelnde Isomaltulose aus der enzymatischen Umsetzung von Saccharose stammen.

Nucleinsäuremoleküle können aus natürlichen Quellen isoliert werden, vorzugsweise aus *Gluconobacter spec.,* oder sie können nach bekannten Verfahren synthetisiert werden. Mittels gängiger molekularbiologischer Techniken ist es möglich, verschiedenartige Mutationen in die erfindungsgemäßen Nucleinsäuremoleküle einzufügen, wodurch es zur Synthese von Enzymen mit eventuell veränderten biologischen Eigenschaften kommt, die ebenfalls von der Erfindung erfasst werden. Mutationen im Sinne der Erfindung betreffen auch alle Deletionsmutationen, die zu verkürzten Enzymen führen. Durch andere molekulare Mechanismen wie Insertionen, Duplikationen, Transpositionen, Genfusion, Nucleotidaustausch aber auch durch Gentransfer zwischen verschiedenen Mikroorganismenstämmen und andere kann es beispielsweise zu Modifikationen der Enzymaktivität und/oder der Regulierung des Enzyms kommen. Auf diese Weise können beispielsweise mutante Enzyme hergestellt werden, die einen veränderten $K_m$-Wert, $K_i$-Wert oder $K_a$-Wert besitzen und/oder den normalerweise in den Zellen vorliegenden Regulationsmechanismen nicht mehr oder in veränderter Form unterliegen. Des weiteren können mutante Enzyme hergestellt werden, die eine veränderte Stabilität, Substrat-, Produktspezifität oder ein verändertes Effektorenmuster oder ein modifiziertes Aktivitäts-, Temperatur-, pH-Wert- und/oder Konzentrations-Profil aufweisen. Weiterhin gibt es Enzyme, die eine veränderte aktive Enzymkonzentration, einen modifizierten Aufbau der Untereinheiten, prä- und posttranslationelle Modifikationen zum Beispiel Signal- und/ oder Transportpeptide und/oder andere funktionelle Gruppen aufweisen.

Es gibt auch Nucleinsäuremoleküle, die mit den vorgenannten erfindungsgemäßen Nucleinsäuremolekülen hybridisieren. Hybridisierung bedeutet eine Hybridisierung unter konventionellen Hybridisierungsbedingungen, wie sie in Sambrook et al. (Molecular Cloning. A laboratory manual, Cold Spring Harbor Laboratory Press, 2. Ausgabe, 1989) beschrieben ist, vorzugsweise unter stringenten Bedingungen. Man spricht von einer Hybridisierung, wenn nach Waschen für eine Stunde mit 1 x SSC und 0,1% SDS bei 55°C, vorzugsweise bei 62°C und besonders bevorzugt bei 68°C, insbesondere für 1 Stunde in 0,2 x SSC und 0,1% SDS bei 55°C, vorzugsweise bei 62°C und besonders bevorzugt bei 68°C noch ein positives Hybridisierungssignal beobachtet wird. Eine unter derartigen Waschbedingungen mit einer der in den Sequenzprotokollen angegebenen Nucleotidsequenzen hybridisierende Nucleotidsequenz ist eine Nucleotidsequenz, die im Zusammenhang mit der Erfindung steht.

Eine Identifizierung und Isolierung derartiger Nucleinsäuremoleküle kann dabei unter Verwendung der erfindungsgemäßen Nucleinsäuremoleküle oder von Teilen dieser Moleküle beziehungsweise des komplementären Stranges erfolgen. Als Hybrisierungsprobe können zum Beispiel Nucleinsäuremoleküle verwendet werden, die exakt die oder im wesentlichen die unter SEQ ID.Nr. 2 dargestellte Nucleotidsequenz oder Teile dieser Sequenz aufweisen. Bei den als Hybridisierungsprobe verwendeten Fragmenten kann es sich auch um synthetische Fragmente handeln, die mit Hilfe üblicher Synthesetechniken hergestellt werden und deren Sequenz im wesentlichen mit der eines Nucleinsäuremoleküls übereinstimmt. Die mit den erfindungsgemäßen Nucleinsäuremolekülen hybridisierenden Moleküle umfassen auch Fragmente, Derivate und allelische Varianten der oben beschriebenen Nucleinsäuremoleküle, die ein erfindungsgemäßes Enzym codieren. Unter "Fragmenten" werden dabei Teile der Nucleinsäuremoleküle verstanden, die lang genug sind, um das beschriebene Enzym zu codieren. Der Ausdruck "Derivat" bedeutet im Zusammenhang mit der Erfindung, dass sich die Sequenzen dieser Moleküle von den Sequenzen der oben beschriebenen Nucleinsäuremoleküle an einer oder mehreren Positionen unterscheiden, aber einen hohen Grad an Homologie zu diesen Sequenzen aufweisen. Homologie bedeutet dabei eine Sequenzidentität von mindestens 40 %, insbesondere eine Identität von mindestens 60 %, vorzugsweise über 80 % und besonders bevorzugt über 90 %, 95 %, 97 % oder 99 % auf Nucleinsäureebene. Dabei weisen die durch diese Nucleinsäuremoleküle codierten Enzyme eine Sequenzidentität zu der in SEQ ID Nr. 1 angegebenen Aminosäuresequenz von mindestens 80 %, vorzugsweise 85 % und besonders bevorzugt von über 90 %, 95 %, 97 % und 99 % auf Aminosäureebene auf. Die Abweichungen zu den oben beschriebenen Nucleinsäuremolekülen können dabei beispielsweise durch Deletion, Substitution, Insertion oder Rekombination entstanden sein. Es kann sich dabei sowohl um natürlicherweise auftretende Variationen handeln, beispielsweise um Sequenzen aus anderen Organismen, oder um Mutationen, wobei diese Mutationen auf natürliche Weise aufgetreten sein können oder durch gezielte Mutagenese (UV- oder Röntgenstrahlen, chemische Agenzien oder andere) eingeführt wurden. Ferner kann es sich bei den Variationen um synthetisch hergestellte Sequenzen handeln. Bei den allelischen Varianten kann es sich sowohl um natürlich auftretende Varianten handeln, als auch um synthetisch hergestellte oder durch rekombinante DNA-Techniken erzeugte Varianten. Die von verschiedenen Varianten der Nucleinsäuremoleküle codierten Enzyme weisen bestimmte gemeinsame Charakteristika auf, wie Enzymaktivität, aktive Enzymkonzentration, Untereinheiten, posttranslationelle Modifikationen, funktionelle Gruppen, Molekulargewicht, immunologische Reaktivität, Konformation und/oder physikalische Eigenschaften, wie das Laufverhalten in Gelelektrophoresen, chromatographisches Verhalten, Sedimentationskoeffizienten, Löslichkeit, spektroskopische Eigenschaften, Stabilität, pH-Wert-Optimum, isoelektrischer pH-Wert, Temperatur-Optimum und/oder andere.

Bei den Nucleinsäuremolekülen kann es sich sowohl um DNA- als auch RNA-Moleküle handeln. Erfindungsgemäße DNA-Moleküle sind beispielsweise genomische DNA oder cDNA-Moleküle.

Ferner gibt es Vektoren, die diese Nucleinsäuremoleküle enthalten. Vorzugsweise handelt es sich dabei um Plasmide, Cosmide, Viren, Bacteriophagen, Shuttle-Vektoren und andere in der Gentechnik übliche Vektoren. Die Vektoren können noch weitere Funktionseinheiten besitzen, die eine Stabilisierung und/oder Replikation des Vektors in einem Wirtsorganismus bewirken oder dazu beitragen.

Es gibt auch Vektoren, bei denen das Nucleinsäuremolekül mit mindestens einem regulatorischen Element operativ verbunden ist, das die Transcription und Synthese translatierbarer Nucleinsäuremoleküle in pro- und/oder eucaryotischen Zellen gewährleistet. Derartige regulatorische Elemente können Promotoren, Enhancer, Operatoren und/oder Transcriptionsterminationssignale sein. Selbstverständlich können die Vektoren auch für ihre Stabilität und/ oder Replikation notwendige Elemente ebenso wie Antibiotikum-Resistenzgene, also Selektionsmarker enthalten.

Die vorgenannten Vektoren können neben der unter Kontrolle mindestens eines regulatorischen Elementes stehenden Nucleinsäuresequenz, welches eine SDH codiert, eine ebenfalls unter der Kontrolle mindestens eines regulatorischen Elementes stehende Nucleinsäuresequenz enthalten, die eine Saccharose-Mutase codiert. Ein derartiger Vektor weist also die genetische Information für die beiden Enzyme auf, die für die enzymatische Umwandlung von Saccharose über Isomaltulose zu 1,6-GPS notwendig sind. Solche Vektoren erlauben es in besonders einfacher Weise, den Stoffwechselapparat eines einzelnen Wirtsorganismusses zu nutzen, um Saccharose enzymatisch in einem Verfahrensschritt zu 1,6-GPS umzuwandeln.

Es gibt auch Wirtszellen, die eines der Nucleinsäuremoleküle oder einen der Vektoren beinhalten beziehungsweise mit diesem transformiert sind und vorzugsweise in der Lage sind, SDH und gegebenenfalls die Saccharose-Mutase zu exprimieren sowie besonders bevorzugt 1,6-GPS aus Isomaltulose beziehungsweise Saccharose herzustellen. Weiterhin sind alle Wirtszellen, die von einer mit den Nucleinsäuremolekülen oder den Vektoren transformierten Wirtszelle abstammen. Also Wirtszellen, die die Nucleinsäuremoleküle oder Vektoren enthalten, wobei unter einer Wirtszelle ein Organismus verstanden wird, der in der Lage ist, *in vitro* rekombinante Nucleinsäuremoleküle aufzunehmen und gegebenenfalls die von den Nucleinsäuremolekülen codierten Enzyme zu synthetisieren. Vorzugsweise sind dies procaryotische oder eucaryotische Zellen. Es gibt vor allem Mikroorganismen, die die Vektoren, Derivate oder Teile der Vektoren enthalten, die diese zu einer Synthese von Enzymen für die Herstellung von 1,6-GPS aus Isomaltulose oder aus Saccharose befähigen. Die Wirtszelle kann auch dadurch gekennzeichnet sein, dass das eingeführte Nucleinsäuremolekül entweder heterolog in bezug auf die transformierte Zelle ist, das heißt natürlicherweise nicht in dieser Zelle vorkommt, oder aber an einem anderen Ort oder einer anderen Kopienzahl im Genom lokalisiert ist als die entsprechende natürlicherweise auftretende Sequenz.

Diese Zelle ist eine procaryotische Zelle, vorzugsweise eine gram-negative procaryotische Zelle, besonders bevorzugt eine Enterobakterienzelle. Dabei kann einerseits eine Zelle verwendet werden, die kein eigenes SDH- und/oder Saccharose-Mutasegen enthält zum Beispiel *E.coli,* andererseits können aber auch Zellen verwendet werden, die bereits ein oder zwei solcher Gene auf ihrem Chromosom enthalten. Bevorzugte Beispiele für geeignete procaryotische Zellen sind *E.coli-, Protaminobacter rubrum-, Erwinia rhapontici-, Enterobacter spec.*- oder *Pseudomonas mesoacidophila-Zellen.* Die Transformation procaryotischer Zellen mit exogenen Nucleinsäuresequenzen ist einem Fachmann auf dem Gebiet der Molekularbiologie geläufig.

Die Zelle kann jedoch auch eine eucaryotische Zelle sein, wie eine Pilzzelle (zum Beispiel Hefe) oder eine tierische Zelle. Verfahren zur Transformation beziehungsweise Transfektion eucaryotischer Zellen mit exogenen Nucleinsäuresequenzen sind dem Fachmann auf dem Gebiet der Molekularbiologie ebenfalls geläufig.

Es gibt auch Zellkulturen, die mindestens eine der Wirtszellen aufweisen, wobei die Zellkultur insbesondere in der Lage ist, eine SDH und gegebenenfalls eine Saccharose-Mutase zu produzieren.

Im Zusammenhang mit der Erfindung steht auch ein Verfahren zur Herstellung einer SDH, wobei die Wirtszelle in einem Kulturmedium unter solchen Bedingungen kultiviert wird, die die Bildung der SDH erlaubt und diese gewonnen werden kann.

Im Zusammenhang mit der Erfindung stehen auch Proteine, die durch die Nucleinsäuremoleküle codiert werden, sowie Verfahren zu deren Herstellung, wobei die Wirtszelle unter Bedingungen kultiviert wird, die die Synthese des Proteins erlaubt, und anschließend das Protein aus den kultivierten Zellen und/oder dem Kulturmedium isoliert wird.

Im Zusammenhang mit der Erfindung stehen auch monoclonale und polyclonale Antikörper, die in der Lage sind, eine Struktur einer erfindungsgemäßen, SDH-Aktivität aufweisenden Einheit zu identifizieren und gegebenenfalls zu binden. Diese Struktur kann ein Protein, Kohlenhydrat wie auch ein Lipidkomplex und/oder Glycolipid sein, die mit der SDHaufweisenden Einheit spezifisch in Beziehung stehen. Darunter sind auch Antikörper, die gegen Strukturen gerichtet sind, die als posttranslationelle Modifikationen der SDH anzusprechen sind. Von Bedeutung ist, dass eine räumliche Elektronenwolkenstruktur mittels Antikörpern so identifiziert werden kann, dass Rückschlüsse auf biologische, chemische und physikalische Eigenschaften der SDH-Aktivität aufweisenden Einheit möglich sind.

Darunter sind auch Antikörper, die mit den vorgenannten Antikörpern interagieren, insbesondere diese identifizieren und binden können.

Darunter sind auch vorgenannte Antikörper, wobei diese auf biologischem, chemischem und physikalischem Wege modifiziert sind.

Die Erfindung soll im folgenden anhand von Beispielen und zugehöriger Figuren erläutert werden.

Die Figuren zeigen:

| | |
|---|---|
| Figur 1 und | eine Plasmidkarte des Plasmids pBtac 2 |
| Figur 2 | eine Plasmidkarte pHWG 467. |
| SEQ ID Nr. 1 | zeigt die 262 Aminosäuren umfassende Aminosäuresequenz der SDH, |
| SEQ ID Nr. 2 | zeigt die 789 Nucleotide umfassende Nucleotidsequenz der SDH, |

SEQ ID Nr. 3 bis 9 zeigen partielle Aminosäuresequenzen der SDH.

**Beispiel 1: Herstellung der SDH**

Die Biomasseproduktion von *Gluconobacter suboxidans*

Um ausreichend Biomasse von *Gluconobacter suboxidans* DSM 2003 zur Verfügung zu stellen, wurde der Stamm im Labormaßstab fermentiert. Als ein Medium 1 wurde verwandt: D-Mannit (50 g/L), Pepton aus Casein (10 g/L), Hefe-Extrakt (5 g/L) und $CaCO_3$ (20 g/L). Ein Medium 2 setzte sich wie folgt zusammen: D-Mannit (75 g/L), Pepton aus Casein (16 g/L), Hefe-Extrakt (8 g/L) und $CaCO_3$ (1 g/L). Als Vorkultur wurde eine gefriergetrocknete Konserve des Stammes *Gluconobacter suboxidans* DSM 2003 in 1 mL Medium 1 suspendiert, auf eine Agar-Platte mit Medium 1 ausgestrichen und 72 h bei 25 °C kultiviert. Daraufhin wurde eine Abimpfung der Agarplatten-Kultur in 25 mL Medium 1 eingebracht und in einem Schüttelkolben 48 h bei 25 °C kultiviert. Die auch als erste Vorkultur bezeichnete Kultur wurde dann in 250 mL Medium 1 in einem Schüttelkolben (Volumen 1 L) für 48 h bei 25 °C kultiviert. Die auf diesem Wege gewonnene - auch als zweite Vorkultur bezeichnete - Kultur diente dann als Inoculum für die Fermentation.

Für die Fermentation wurde die zweite Vorkultur aus dem Schüttelkolben in 15 L des Mediums 2 in einen 20 L Fermenter transferiert und bei 25 °C unter folgenden Bedingungen kultiviert:

pH-Wert: 6,9 (konstant gehalten durch Titration mit 5 N NaOH)
Rührgeschwindigkeit: 350 U/min und
Sauerstoff-Sättigung: 40 %.

Die genannten Bedingungen wurden während der Fermentation konstant gehalten. Die Fermentation wurde mit dem Ende des Zellwachstums - der durch Substratverbrauch per HPLC festgestellt wurde - beendet.

Die so gewonnene Zellmasse wurde durch Zentrifugation (8000 x g, 20 min, 4 °C) geerntet und mit einer 0,9 %-igen NaCl-Lösung gewaschen und in 50 mM Tris-Puffer (pH 7,0) 1:3 verdünnt und folgend im French-Press-Verfahren aufgeschlossen. Die entstandenen Zelltrümmer wurden durch Ultrazentrifugation (110.000 x g, 20 min, 4 °C) entfernt. Der klare Überstand - der sogenannte Rohextrakt - wurde sofort eingesetzt oder bei -30 °C gelagert.

Reinigung der SDH

Für die Reinigung der SDH wurde das folgende Drei-Schritt-Schema gewählt:

Schritt 1:

Der Rohextrakt wurde auf eine mit 50 mM Tris/HCl-Puffer (pH 7,0) äquilibrierte DEAE-Sepharose-Säule (2,6 x 9 cm) aufgetragen und mit einem linearen Gradienten von 0,0 - 0,5 M NaCl in 50 mM Tris/HCl-Puffer (pH 7,0) und bei einer Flußrate von 50 cm/h eluiert. Die SDH konnte bei 0,2 M NaCl eluiert werden.

Schritt 2:

Eine Farb-Liganden-Affinitätschromatographiesäule wurde wie folgt vorbereitet: (i) ein Sepharose 4B-CL Gel wurde als Matrix eingesetzt (Boyer, P.M. und Hsu, J.T. (1993) In: Fiechter, A. (ed.), Advances in Biochemical engineering, 49, 1-44) und (ii) der Farbstoff Blue 160 (Blue H-ERD) wurde nach der Methode Neuhauser, W. et al., 1997 (Biochem. J., 326, 683-692) immobilisiert. Dazu wurde 10 mg Farbstoff pro 1 mL Gel eingesetzt. Die gepoolten SDH-haltigen Fraktionen aus Schritt 1 wurden dialysiert und auf die Farb-Liganden-Säule (2,6 x 7,0 cm, äquilibriert mit 50 mM Tris/HCl-Puffer, pH 7,0) aufgetragen (ca. 2 - 5 mg Protein/mL Gel). Das adsorbierte Protein wurde mit einem linearen Gradienten von 0,0 - 0,5 M NaCl in 50 mM Tris/HCl-Puffer, pH 7,0 und einer Flußrate von 2,0 mL/min eluiert. Die SDH konnte bei 0,1 M NaCl eluiert werden.

Schritt 3:

Die gepoolten, aktiven Fraktionen aus Schritt 2 wurden dialysiert und durch Ultrafiltration aufkozentriert und nochmals auf eine Farb-Liganden-Säule aufgetragen (2,6 x 7,0 cm, äquilibriert mit 50 mM Tris/HCl-Puffer, pH 7,0), wobei ca. 0,5 mg Protein auf 1 mL Gel aufgetragen wurden. Die Affinitätselution der reinen SDH wurde mit 10 mM NADH in 20 mM Tris/HCl-Puffer, pH 7,0, bei einer Flußrate von 10 cm/h durchgeführt. Die NADH wurde anschließend durch Dialyse entfernt.

Die gereinigte SDH kann bei -30 °C für ca. 8 Monate gelagert werden, bei +4 °C ist die SDH für ca. 30 Tage stabil.

Tabelle 1. Reinigungsschema für die SDH

| Reinigungsschritt | Gesamt-Aktivität | Gesamt-Protein | spezifische Aktivität | Anreicherung | Ausbeute |
|---|---|---|---|---|---|
| | (U) | (mg) | ($U.mg^{-1}$) | (fach) | (%) |
| Rohextrakt | 360,0 | 782,6 | 0,46 | 1,0 | 100 |
| DEAE-Sepharose | 281,0 | 78,5 | 3,58 | 7,8 | 78 |
| Blue 160-Affigel | 174,0 | 15,7 | 11,12 | 24,2 | 48 |
| Blue 160-Affigel | 120,0 | 4,9 | 24,49 | 53,2 | 33 |

Mittels des dargestellten Reinigungsschemas, also Separation des Rohextraktes mittels Anionentauscherchromatographie, anschließender erster Farb-Liganden-Affinitätschromatographie, Ultrafiltration und zweiter Farb-Liganden-Affinitätschromatographie, kann eine Anreicherung um das ungefähr 50fache gegenüber dem Wert des Rohextraktes erreicht werden. Die Ausbeute beträgt dann ca. 33 %.

**Beispiel 2: Herstellung von 1,6-GPS aus Isomaltulose**

Die enzymatische, kontinuierliche Herstellung von 1,6-GPS in einem Enzym-Membran-Reaktor

Mit einem Edelstahlreaktor (Volumen 50 ml) wurde die kontinuierliche Herstellung von 1,6-GPS aus Isomaltulose durchgeführt. Der Enzym-Membran-Reaktor besaß einen Doppelmantel zur Temperierung mittels Wasserkühlung, Bohrungen zum Einsatz einer Leitfähigkeits- und pH-Wert-Elektrode und Anschlüsse zur Druckmessung, Substratzufuhr, Produktabfuhr und Probenentnahme. Der im Reaktorinnenraum befindliche Magnetrührer, der extern betrieben werden kann, garantiert die optimale Durchmischung der eingesetzten Substanzen. Der Deckel des Reaktors enthielt auf der Unterseite eine Metallsinterplatte, auf welcher eine Ultrafiltrationsmembran und ein O-Ring angebracht waren. Nach dem Befüllen des Reaktors wurde der Deckel mit dem Unterteil verschraubt. Die Zuführung des Substrates erfolgte mit einer Kolbenpumpe. Die Leitfähigkeit und der pH-Wert der Reaktionslösung wurden kontinuierlich und direkt gemessen, wobei ein Regelsystem den pH-Wert titrimetrisch konstant hielt. Da Ultrafiltrationsmembranen keine völlige Rückhaltung des Coenzyms gewährleisten, wurde eine geladene Membran der Firma Nitto (Nitto-Membran NTR-7410) eingesetzt. Diese elektrostatische Methode der Coenzymrückhaltung garantiert den Verbleib auch von dissoziierten Coenzymen im gewünschten Reaktionsraum.

Der beschriebene Membran-Reaktor wurde mit einer Lösung aus 250 mM Isomaltulose und Glucose in 50 mM Tris/HCl-Puffer pH 7,5 (+0,1 % $NaN_3$) befüllt. Bei einem Fluss von 2 ml/h wurde der Reaktor anschließend 24 Stunden im "Leerlauf" betrieben, um eventuelle Undichtheiten oder andere Probleme zu erkennen und gegebenenfalls zu beseitigen. Anschließend wurde SDH und das Regenerations-Enzym FDH mit einen Superloop, wie er zum Probenauftrag in der Chromatographie verwendet wird, eingebracht. Zu Beginn der Reaktion wurden je 1 U/ml von vollständig gereinigter SDH (aus Beispiel 1) und GDH (Glucose-Dehydrogenase aus *Bacillus megaterium)* eingesetzt. Durch Injektion von 2,5 $\mu$M $NAD^+$ erfolgte der Start der Reaktion. Der pH-Wert wurde durch Titration mit 1M Tris auf pH 7,0 konstant gehalten. Eine wichtige Größe beim Betrieb eines kontinuierlichen Reaktors ist die Verweilzeitverteilung. Diese errechnet sich unter der Annahme von optimaler Durchmischung der Reaktionslösung nach der Formel

$$\tau = \frac{}{V_0},$$

wobei V das Reaktorvolumen und $V_0$ der Volumenstrom sind. Nach vier Stunden Batch-Versuch wurde die Substratzufuhr auf den Fluss von 2 ml/h eingestellt, so dass die mittlere Verweilzeit ($\tau$) 25 Stunden betrug. Die Versuchsdauer betrug 234 Stunden, wobei in regelmäßigen Abständen die Enzymaktivitäten, Laugeverbrauch, Leitfähigkeit, Druck, Substrat und Produktvolumen bestimmt wurden. Die erhaltenen Produktproben wurden mittels HPLC auf ihre Zusammensetzung hin untersucht und die Bildung von 1,6-GPS nachgewiesen.

**Beispiel 3: Enzymatisches Ein-Bioreaktor-Verfahren ("Eintopfreaktion") zur Herstellung von 1,6-GPS aus Saccharose**

Zur direkten enzymatischen Herstellung von 1,6-GPS aus Saccharose wurde ein Batch-Versuch mit immobilisierten Zellen von P. *rubrum* sowie dem Enzym SDH aus *G. suboxidans* 2003 und FDH aus *Candida boidinii* unter nachstehend aufgeführten Bedingungen durchgeführt. Die Substrate in dieser Reaktion waren Saccharose und Ammoniumformiat.

| | |
|---|---|
| Volumen: | 10 ml |
| Temperatur: | 25 °C |
| Saccharose: | 500 mM |
| Formiat: | 500 mM |
| P. *rubrum* Zellen: | 1 g |
| SDH | 5 U |
| FDH: | 10 U |
| NAD: | 1 mM |

Da bei dieser Reaktion der pH-Wert anstieg, wurde mit 1 M Ameisensäure konstant auf pH-Wert 7,0 geregelt. Der Substratsverbrauch wurde während der Reaktion mittels DC- und DNS-Methoden (für den Umsatz von Saccharose zu Isomaltulose) sowie HPLC (für den Umsatz von Isomaltulose zu 1,6-GPS) kontrolliert. Es konnte die direkte Bildung von 1,6-GPS innerhalb eines Reaktionsgefäßes und in einem Verfahrensschritt aus Saccharose gezeigt werden.

**Beispiel 4: Nucleinsäure und abgeleitete Peptidsequenz der SDH.**

1. Peptidsequenzermittlung

Zur Ermittlung von Teilaminosäuresequenzen wurde die gemäß Beispiel 1 gereinigte SDH von *Gluconobacter suboxidans* DSM 2003 tryptisch verdaut und nach Standardmethoden getrennt. Die erhaltenen partiellen Peptidsequenzen sind in SEQ ID Nr. 3 bis 9 dargestellt.

2. DNA-Sonden-Bereitstellung

Basierend auf den bei der Peptidsequenzermittlung analysierten Sequenzen wurden Primer hergestellt, mit deren Hilfe die Clonierung des gesamten Gens der SDH aus *Gluconobacter suboxidans* DMS 2003 gelang.

3. Erstellung der Genbank und Clonisolierung

Die vorgenannten Primer (SEQ ID Nr. 3 und 5) wurden in einer PCR-Reaktion eingesetzt und ein Fragment der SDH-codierenden Sequenz erhalten. Mit dem erhaltenen PCR-Fragment wurde eine DNA-Sonde hergestellt. Im genomischen Southern-Blot konnten nach Markierung der Sonde hybridisierende Banden festgestellt werden. Es wurden zwei unabhängige subgenomische Genbanken (nach ClaI Verdau ~9kb Fragment, nach BamHI Verdau ~23,5 kb Fragment, Reinigung der Fragmente über Saccharosegradientenzentrifugation) mit gereinigten Fragmentscharen im pBlueskrip SK erstellt. Mit der erwähnten markierten Sonde wurden die subgenomischen Clonbanken durch Hybridisierung geprüft. Es gelang, die Identifizierung mehrerer Clone, von denen je ein Clon aus der BamHI- und ClaI-Bank weiter analysiert wurden.

4. Sequenzanalyse

Die Sequenzermittlung der clonierten Fragmente ergab in beiden Fällen eine übereinstimmende DNA-Sequenz. Aus dieser konnte die Aminosäuresequenz der SDH erstellt werden. Sie stimmt mit den Sequenzen aus den Peptidsequenzen überein, bis auf die Position Codon 196, für die die Peptidsequenz für Peptid 5 Serin (S) statt dem nun ermittelten Tryptophan (W 196) ergeben hatte.

Die Nucleotidsequenz der SDH von *Gluconobacter suboxidans* DSM 2003 ist unter SEQ ID Nr. 2 dargestellt und umfasst 789 Nucleotide

Die Aminosäuresequenz der Sorbitol-Dehydrogenase von *Gluconobacter suboxidans* DSM 2003 ist dargestellt unter SEQ ID Nr. 1 und umfasst 262 Aminosäuren.

5. Expressionen in *E. coli*

Die codierende Sequenz wurde am 5'-Ende mit der Sequenz GAATTCTT und am 3'-Ende mit der Sequenz AAGCTT verlängert. Diese Sequenz wurde in den Vektor pBtac2 (Fig. 1; J. Bosius (1988), Biotechnology 10, 205-225) der mit *Eco*RI und *Hind*III vorbereitet worden war, integriert. Das resultierende Plasmid pHWG 467 (Fig. 2) enthält ein Ampicillin-Resistenzgen und die SDH-codierende Sequenz unter Kontrolle des tac-Promoters. Plasmid pHWG 467 wurde in *E.coli* JM109 transformiert. Diese Zellen produzieren nach Induktion mit IPTG SDH-Aktivität. Das entsprechende Protein kann in Rohextrakten in seiner Aktivität bestimmt werden. Im SDS-Gel dieser Extrakte ist das Protein als dominante Bande bei 28 kDa mit Coomassie Brilliant Blue färbbar.

## Patentansprüche

1. Verfahren zur enzymatischen Herstellung von 6-O-α-D-Glucopyranosyl-D-sorbit (1,6-GPS) aus Isomaltulose, wobei die Isomaltulose in eine wäßrige Reaktionslösung enthaltend eine Sorbitol-Dehydrogenase (SDH)-Aktivität aufweisende Einheit gegeben, inkubiert und anschließend 1,6-GPS aus der Reaktionslösung gewonnen wird.

2. Verfahren nach Anspruch 1, wobei der wäßrigen Reaktionslösung vor oder während der Inkubation Reduktionsäquivalente zugegeben werden.

3. Verfahren nach Anspruch 1, wobei die SDH-Aktivität aufweisende Einheit isolierte SDH ist.

4. Verfahren nach Anspruch 1, wobei die SDH-Aktivität aufweisende Einheit ein SDH enthaltender lebender oder toter Mikroorganismus oder ein Rohextrakt davon ist.

5. Verfahren nach Anspruch 4, wobei der SDH enthaltende Mikroorganismus Gluconobacter suboxidans DSM 2003 ist.

6. Verfahren nach einem der Ansprüche 1 bis 5, wobei das Verfahren mit oder ohne pH-Wert-Regulierung durchgeführt wird.

7. Verfahren nach einem der Ansprüche 1 bis 6, wobei der wäßrigen Reaktionslösung vor oder während der Inkubation ein Regenerationsmittel, insbesondere Formiat-Dehydrogenase (FDH) und Formiat, zugegeben wird.

8. Verfahren nach einem der Ansprüche 3 bis 7, wobei der Mikrorganismus, der Rohextrakt oder das Enzym immobilisiert ist.

9. Verfahren nach einem der Ansprüche 1 bis 8, wobei das Verfahren kontinuierlich durchgeführt wird.

10. Verfahren nach einem der Ansprüche 1 bis 9, wobei die als Ausgangsmaterial eingesetzte Isomaltulose durch enzymatische Umsetzung von Saccharose zu Isomaltulose hergestellt wird.

11. Verfahren nach Anspruch 10, wobei die enzymatische Umsetzung von Saccharose zu Isomaltulose mittels immobilisierter P. rubrum-Zellen, isolierter Saccharose-Mutase und/oder einem Zellaufschluß von P. rubrum durchgeführt wird.

12. Verfahren nach Anspruch 10 oder 11, wobei die enzymatische Umsetzung von Saccharose zu Isomaltulose und von Isomaltulose zu 1,6-GPS in einem einzigen Verfahrensschritt erfolgt.

13. Verfahren nach einem der Ansprüche 1 bis 12, wobei die Umsetzung von Saccharose zu Isomaltulose und von Isolmaltulose zu 1,6-GPS in einem einzigen Bioreaktor durchgeführt wird.

14. Verfahren nach einem der Ansprüche 1 bis 13, wobei die enzymatische Herstellung bei Temperaturen von 20°C bis 40°C, insbesondere 25°C stattfindet.

15. Verfahren zur enzymatischen Herstellung von 1,6-GPS aus Saccharose, wobei Saccharose enzymatisch zu Isomaltulose umgewandelt und anschließend oder gleichzeitig die Isomaltulose enzymatisch mittels eines Verfahrens nach einem der Ansprüche 1 bis 14 zu 1,6-GPS umgesetzt wird.

## Claims

1. Method for enzymatic preparation of 6-O-α-D-glucopyranosyl-D-sorbitol (1,6-GPS) from isomaltulose, wherein the isomaltulose is put into an aqueous reaction solution containing a unit having sorbitol dehydrogenase (SDH) activity, incubated, and then 1,6-GPS is recovered from the reaction solution.

2. Method according to claim 1, wherein reduction equivalents are added to the aqueous reaction solution before or during the incubation.

3. Method according to claim 1, wherein the unit having SDH activity is isolated SDH.

4. Method according to claim 1, wherein the unit having SDH activity is a living or dead microorganism containing SDH or a raw extract thereof.

5. Method according to claim 4, wherein the microorganism containing SDH is Gluconobacter suboxidans DSM 2003.

6. Method according to one of claims 1 to 5, wherein the method is carried out with or without pH value regulation.

7. Method according to one of claims 1 to 6, wherein a regeneration agent, particularly formate dehydrogenase (FDH) and formate, is added to the aqueous reaction solution before or during the incubation.

8. Method according to one of claims 3 to 7, wherein the microorganism, the raw extract or the enzyme is immobilized.

9. Method according to one of claims 1 to 8, wherein the method is carried out continuously.

10. Method according to one of claims 1 to 9, wherein the isomaltulose used as starting material is prepared by enzymatic conversion of sucrose to isomaltulose.

11. Method according to claim 10, wherein the enzymatic conversion of sucrose to isomaltulose is carried out by means of immobilized P. rubrum cells, isolated sucrose mutase and/or a cell digest of P. rubrum.

12. Method according to claim 10 or 11, wherein the enzymatic conversion of sucrose to isomaltulose and of isomaltulose to 1,6-GPS takes place in a single process step.

13. Method according to one of claims 1 to 12, wherein the conversion of sucrose to isomaltulose and of isomaltulose to 1,6-GPS is carried out in a single bioreactor.

14. Method according to one of claims 1 to 13, wherein the enzymatic preparation takes place at temperatures from 20°C to 40°C, particularly 25°C.

15. Method for enzymatic preparation of 1,6-GPS from sucrose, wherein sucrose is enzymatically converted to isomaltulose and subsequently or simultaneously the isomaltulose is enzymatically converted to 1,6-GPS by means of a method as in one of claims 1 to 14.

## Revendications

1. Procédé de fabrication enzymatique de 6-O-α-D-glucopyranosyl-D-sorbitol (1,6-GPS) à partir d'isomaltulose, dans lequel l'isomaltulose est versé dans une solution réactionnelle aqueuse contenant une unité présentant une activité de sorbitol-déshydrogénase (SDH), est laissé à incubation, puis le 1,6-GPS est extrait de la solution réactionnelle.

2. Procédé selon la revendication 1, dans lequel à la solution réactionnelle aqueuse sont ajoutés des équivalents de réduction avant ou pendant l'incubation.

3. Procédé selon la revendication 1, dans lequel l'unité présentant une activité de SDH est de la SDH isolée.

4. Procédé selon la revendication 1, dans lequel l'unité présentant une activité de SDH est un microorganisme vivant ou mort contenant de la SDH ou un extrait brut de celui-ci.

5. Procédé selon la revendication 4, dans lequel le microorganisme contenant de la SDH est Gluconobacter suboxidans DSM 2003.

6. Procédé selon l'une quelconque des revendications 1 à 5, dans lequel le procédé est effectué avec ou sans régulation de la valeur du pH.

7. Procédé selon l'une quelconque des revendications 1 à 6, dans lequel à la solution réactionnelle aqueuse est ajouté avant ou pendant l'incubation un régénérant, en particulier du formiate déshydrogénase (FDH) et du formiate.

8. Procédé selon l'une quelconque des revendications 3 à 7, dans lequel le microorganisme, l'extrait brut ou l'enzyme est immobilisé.

9. Procédé selon l'une quelconque des revendications 1 à 8, dans lequel le procédé est effectué en continu.

10. Procédé selon l'une quelconque des revendications 1 à 9, dans lequel l'isomaltulose utilisé comme matière de départ est fabriqué par transformation enzymatique de saccharose en isomaltulose.

11. Procédé selon la revendication 10, dans lequel la transformation enzymatique de saccharose en isomaltulose est effectuée au moyen de cellules de P. rubrum immobilisées, de saccharose mutase isolée et/ou d'un fractionnement cellulaire de P. rubrum.

12. Procédé selon la revendication 10 ou 11, dans lequel la transformation enzymatique de saccharose en isomaltulose et d'isomaltulose en 1,6-GPS s'effectue en une seule étape de procédé.

13. Procédé selon l'une quelconque des revendications 1 à 12, dans lequel la transformation de saccharose en isomaltulose et d'isomaltulose en 1,6-GPS est effectuée dans un seul bioréacteur.

14. Procédé selon l'une quelconque des revendications 1 à 13, dans lequel la fabrication enzymatique a lieu à des températures de 20°C à 40°C, en particulier à 25°C.

15. Procédé de fabrication enzymatique de 1,6-GPS à partir de saccharose, dans lequel du saccharose est converti enzymatiquement en isomaltulose et dans lequel, ensuite ou simultanément, l'isomaltulose est transformé enzymatiquement au moyen d'un procédé selon l'une des revendications 1 à 14 en 1,6-GPS.

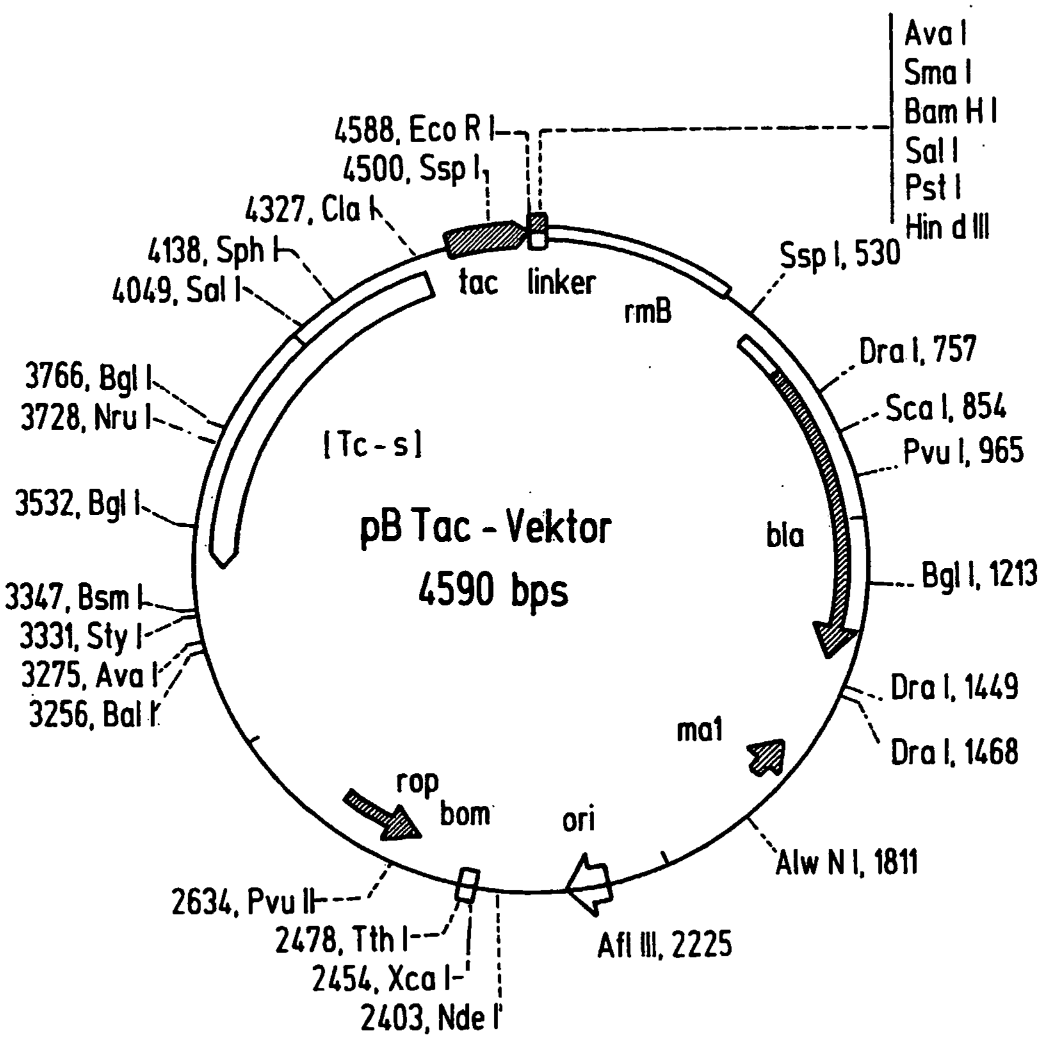
Ava I
Sma I
Bam H I
Sal I
Pst I
Hin d III
4588, Eco R I
4500, Ssp I
4327, Cla I
4138, Sph I
4049, Sal I
tac
linker
rrnB
Ssp I, 530
Dra I, 757
Sca I, 854
Pvu I, 965
3766, Bgl I
3728, Nru I
[Tc-s]
3532, Bgl I
pB Tac - Vektor
4590 bps
bla
Bgl I, 1213
3347, Bsm I
3331, Sty I
3275, Ava I
3256, Bal I
Dra I, 1449
Dra I, 1468
ma1
rop
bom
ori
Alw N I, 1811
2634, Pvu II
2478, Tth I
2454, Xca I
2403, Nde I
Afl III, 2225

Fig.1

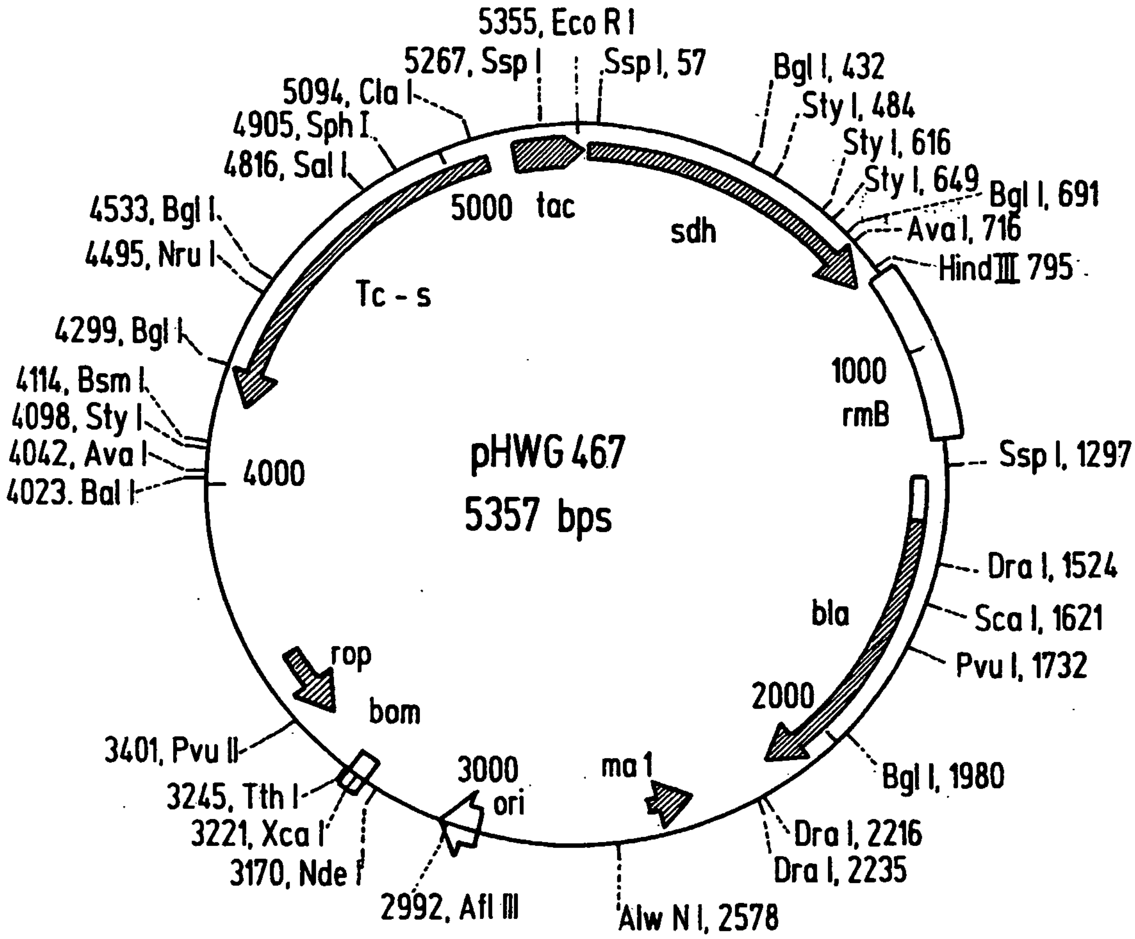
5355, Eco R I
5267, Ssp I
Ssp I, 57
5094, Cla I
4905, Sph I
4816, Sal I
4533, Bgl I
4495, Nru I
4299, Bgl I
4114, Bsm I
4098, Sty I
4042, Ava I
4023. Bal I
Bgl I, 432
Sty I, 484
Sty I, 616
Sty I, 649
Bgl I, 691
Ava I, 716
HindIII 795
Ssp I, 1297
Dra I, 1524
Sca I, 1621
Pvu I, 1732
Bgl I, 1980
Dra I, 2216
Dra I, 2235
Alw N I, 2578
2992, Afl III
3170, Nde I
3221, Xca I
3245, Tth I
3401, Pvu II
5000
tac
sdh
Tc - s
1000
rmB
4000
pHWG 467
5357 bps
bla
2000
rop
bom
3000
ori
ma 1

Fig.2

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 4414185 C1
- DE 19701439 A1
- DE 19705664 A1
- DE 19523008 A1
- EP 0625578 B1
- EP 9500165 W
- DE 19523560 A1


### In der Beschreibung aufgeführte Nicht-Patentliteratur


- **SCHIWECK.** *alimenta,* 1980, vol. 19, 5-16
- **SAMBROOK et al.** Molecular Cloning. A laboratory manual. Cold Spring Harbor Laboratory Press, 1989
- **BOYER, P.M. ; HSU, J.T.** Advances in Biochemical engineering. 1993, vol. 49, 1-44
- **NEUHAUSER, W. et al.** *Biochem. J.,* 1997, vol. 326, 683-692
- **J. BOSIUS.** *Biotechnology,* 1988, vol. 10, 205-225